# EUROPÄISCHE PATENTSCHRIFT

(11) **EP 4 288 018 B1**
(45) Veröffentlichungstag und Bekanntmachung des Hinweises auf die Patenterteilung: **18.06.2025**
(21) Anmeldenummer: 22701535.1
(22) Anmeldetag: 11.01.2022
(51) Int. Cl.: A61K 8/04, A61K 8/26, A61K 8/33, A61K 8/37, A61Q 15/00

(54) **ANTITRANSPIRANTSPRAYS MIT GERINGERER TEXTILVERFLECKUNG**
ANTIPERSPIRANT SPRAY HAVING A LOWER TENDENCY TO STAIN TEXTILES
SPRAY ANTI-TRANSPIRANT AYANT UNE TENDANCE RÉDUITE À LA FORMATION DE TACHES SUR LES TEXTILES

(30) Priorität: 02.02.2021 DE 102021200919
(43) Veröffentlichungstag der Anmeldung: 13.12.2023
(73) Patentinhaber: Beiersdorf AG, 22529 Hamburg (DE)
(72) Erfinder: SCHOLZ, Annika, 22844 Norderstedt (DE); MIERTSCH, Heike, 22459 Hamburg (DE); IBISI, Elita, 22117 Hamburg (DE)
(74) Vertreter: Beiersdorf AG
(86) Internationale Anmeldenummer: PCT/EP2022/050428
(87) Internationale Veröffentlichungsnummer: WO 2022/167176

(56) Entgegenhaltungen:
- DE-A1- 102017 223 179
- FR-A1- 3 046 056
- DATABASE GNPD [online] MINTEL; 5 June 2020 (2020-06-05), ANONYMOUS: "Foot Perfume", XP055915938, retrieved from https://www.gnpd.com/sinatra/recordpage/7755715/ Database accession no. 7755715

## Beschreibung

Die Erfindung gehört zum kosmetischen Bereich und betrifft antitranspirant wirksame Aerosolsprays mit verminderter Textilverfleckung.

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der Haut eingesetzt.

Eine Kategorie von Kosmetikprodukten sind Deodorantien und Antitranspirantien, welche unter den Achseln angewendet werden, um Schlechtgerüche zu vermeiden. Ursache der Schlechtgerüche ist die Zersetzung von an sich geruchlosem Schweiß durch Bakterien. Als Produkt bei der Zersetzung treten Fettsäurereste auf, die den typisch bekannten Schweißgeruch charakterisieren. Unabhängig davon, dass Deodorantien und Antitranspirantien auf dem gleichen Hautareal angewendet werden und dieselbe Zielsetzung verfolgen, unterscheiden sie sich in ihrer technischen Wirkung.

Antitranspirantien enthalten Antitranspirantwirkstoffe, die dazu dienen den Schweißfluss zu behindern und zu reduzieren. Bei aluminiumhaltigen Antitranspirantwirkstoffen kommt es zu einer Verstopfung der Schweißdrüsen.

Deodorantien sind hingegen dadurch gekennzeichnet, dass diese den Schweißfluss nicht hindern. Stattdessen werden unter Deodorantien Produkte verstanden die spezielle antimikrobielle Wirkstoffe enthalten, welche das Wachstum von Bakterien, wie *Corynebacterium jeikeium* oder *Staphylococcus epidermidis,* reduzieren. Folglich wird die Zersetzung des geruchslosen Schweißes vermindert, so dass weniger Schlechtgerüche auftreten.

Konventionell werden Antitranspirantien und Deodorantien entweder mittels eines Applikationsrollers oder als Spray auf die Haut aufgetragen. Als Spray kommen sowohl Treibgas betriebene Aerolsolsprays als auch mittels einer Handpumpe betriebene Pumpsprays zum Einsatz. Abhängig von der Applikationsform variieren die kosmetischen Zubereitungen signifikant in ihrer Zusammensetzung.

Bei Anwendung von Antitranspirantien oder Deodorantien reklamieren viele Verbraucher unerwünschte Flecken im Achselbereich in der Kleidung. Es handelt sich dabei häufig um gelbliche Flecken, die auch zu Verkrustungen neigen können. Diese Ablagerungen und Flecken entstehen vor allem durch ein komplexes Zusammenspiel zwischen Produkt, Hautfett, Sebum, Schweiß und Waschmittel und lassen sich durch herkömmliche Reinigungsverfahren nur schwer entfernen.

Die Flecken können je nach Person unterschiedlich ausgeprägt sein. Eine Ursache sind die in den meisten Deo-AT-Produkten eingesetzten Aluminiumsalze, die als Antitranspirantwirkstoffe fungieren. Diese hartnäckigen Verfärbungen lassen sich beim Waschen, auch bei Vorbehandlungen mit Fleckenmittel, nicht oder nur schwer vollständig entfernen.

Es gibt zahlreiche Literatur und Patente, die sich mit der Fleckenbildung auf der Haut und der Kleidung und deren Vermeidung bei der Anwendung von Antitranspirantien befassen.

In der EP 1178775 A1 wird die Verwendung von wasserlöslichen Tensiden zur Verbesserung der Abwaschbarkeit der Rückstände von der Haut und Kleidung beschrieben. Es werden Kombinationen von adstringierenden Salzen mit wasserlöslichen, nichtionischen Tensiden beschrieben, die einen raschen Antitranspirantwirkungseintritt und eine hohe Effektivität aufweisen sollen.

Die EP 973492 A1 beschreibt die Verwendung von oberflächenaktiven Substanzen, hauptsächlich nichtionische Emulgatoren, in Antitranspirantien. Die Antitranspirantstiftformulierungen umfassen nichtflüchtige Emollients, einen Träger, z.B. Cyclomethicon, einen Fettalkohol, wie Stearylalkohol, ein Antitranspirantmaterial und ein Tensid. Es wird die Problematik der Bildung von weißen Rückständen auf der Haut und der Kleidung, die sich bei Anwendung von aluminiumhaltigen Antitranspirantien bilden können, beschrieben. Hierin wird das Problem des Weißelns der Formulierung durch einen Refraktionsindexabgleich der Bestandteile angegangen.

EP 858317 A1 beschreibt Zubereitungen mit oberflächenaktiven Substanzen mit einem HLB > 10 zur Entfernung der fettigen Rückstände auf der Haut.

EP 696188 A1 beschreibt den Einsatz eines wash-off Agents zur Entfernung der Lipidkomponenten von der Haut, bevorzugt werden dafür Ethoxylate eingesetzt.

Die DE 102008052748, eine frühere Anmeldung der Patentinhaberin, beschreibt, dass in wasserfreien Suspensionen Emulgatoren zur verbesserten Abwaschbarkeit der Formulierung von der Haut eingesetzt werden. Die in der wasserfreien Formel vorteilhaft enthaltenen Strukturgeber können auf der Haut fühlbar wachsige Rückstände hinterlassen. Durch die Anwesenheit von polaren Gruppen an den eingesetzten Emulgatoren wird beim Abwaschen der Formulierung die Affinität zum Wasser erhöht und die Rückstände verschwinden. Dafür eignen sich bevorzugt nichtionische Emulgatoren. Ein weiteres Beispiel bildet DE102017 223179A1.

Antitranspirant wirksame Zubereitungen, die mittels eines Treibmittels aus einer Aerosoldose versprüht werden, liegen zumeist als wasserfreie Suspension vor. Eine wasserfreie Suspension bedeutet, dass dieser Suspension kein freies Wasser zugesetzt wurde. Kristallwasser, Hydratationswasser oder ähnlich molekular gebundenes Wasser kann jedoch vorhanden sein. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist beispielsweise solches Wasser, das als Lösemittel, als Gelaktivator oder als Lösemittelbestandteil anderer Wirkstoffe zur Suspension zugegeben werden würde. In diesem Fall wäre die Suspension nicht mehr wasserfrei. Die wasserfreien Suspensionen für Aerosolsprays enthalten neben schweißreduzierenden Wirkstoffen in der Regel mindestens ein kosmetisches Öl als Träger für den teilchenförmigen schweißreduzierenden Wirkstoff. Die Suspensionen werden in einem druckfesten Behälter, meist einer Dose aus Weißblech oder Aluminium, die innen lackiert ist, zusammen mit einem verflüssigten Kohlenwasserstoff, wie n-Butan, iso-Butan und/oder Propan, als Treibmittel abgefüllt. Vor Gebrauch des Sprühventils, bei dem Treibmittel und eine Portion der Suspension freigesetzt werden, muss der Behälter zunächst ausreichend geschüttelt werden, um den abgesetzten Antitranspirantwirkstoff aufzumischen. Damit sich der suspendierte Antitranspirantwirkstoff nicht sofort wieder absetzt, enthalten handelsübliche Suspensionen ein Suspendiermittel, z. B. hydrophob modifizierte Hectorite, wie sie beispielsweise unter der Handelsbezeichnung Bentone Gel von den Firmen Rheox und Elementis Specialties erhältlich sind, oder auch hydrophile und/oder hydrophob modifizierte Kieselsäuren.

Cyclomethicone ist im Stand der Technik bereits als gut geeignetes Trägeröl für Antitranspirantsprays bekannt. Da dieses Öl eine relativ hohe Flüchtigkeit aufweist und dadurch den Antitranspirantwirkstoff nicht zu stark blockiert, erzielt man mit Cyclomethicone eine sehr zufrieden stellende Freisetzung des Antitranspirantwirkstoffs sowie eine relativ geringe Textilanschmutzung. Jedoch sollte der Einsatz von Cyclomethicone sowie anderer Silikonöle aufgrund seiner Persistenz möglichst vermieden werden.

WO 2010097205 A1 offenbart in den Beispielen P10, 12,13 und 14 antitranspirant wirksame Zubereitungen, welche dafür vorgesehen sind, mittels eines Treibmittels aus einer Aerosoldose zur Applikation auf die Haut ausgetragen zu werden. Die Zusammensetzungen enthaltend zu großen Teilen Silikonöle. Gleichzeitig adressiert WO 2010097205 A1 die Aufgabe, kosmetische Antitranspirantien zur Verfügung zu stellen, die eine verringerte Fleckenbildung in der Kleidung aufweisen und vor allem die nachträgliche Auswaschbarkeit verbessern helfen. Gelöst wurde die Aufgabe durch den Zusatz von ein oder mehr Tensiden, insbesondere durch den Zusatz von Palmitamidopropyltrimoniumchlorid.

WO 2013092208 A2 betrifft ebenfalls antitranspirant wirksame Zubereitungen, welche dafür vorgesehen sind mittels eines Treibmittels aus einer Aerosoldose zur Applikation auf die Haut ausgetragen zu werden. Ferner adressiert WO 2013092208 A2 die effektive Freisetzung des schweisshemmenden Wirkstoffs aus einer wasserfreien Antitranspirant-Zusammensetzung sowie die Verminderung der Textilanschmutzungen. Dabei soll der Einsatz von Cyclomethicone in der Zusammensetzung vermindert werden, obwohl der Einsatz von Cyclomethicone zu einer geringeren Textilanschmutzung führt. Die Zusammensetzungen aus WO 2013092208 A2 nutzen als Ersatz eine Mischung aus hohen Anteilen von nicht flüchtigen Silikonölen in Kombination mit einem niedrigen Anteil von nichtflüchtigen Ölen und mindestens einem Paraffinöl (Mineralöl) sowie 0 bis weniger als 1 Gew.-% Cyclomethicone.

Nachteilig an WO 2013092208 A2 ist der Umstand, dass eine geringeren Textilanschmutzung nur durch den Einsatz von Silikonölen sowie Mineralölen, wie Paraffinöl, ermöglicht wird. Beim Verbraucher besteht jedoch zunehmend der Wunsch neben Silikonölen auch auf Mineralöle, wie Paraffinöle, zu verzichten, da diese nicht als natürlich wahrgenommen werden. Zudem stehen Mineralöle oftmals im Verdacht Verunreinigungen von unerwünschten MOSH (steht für Englisch "Mineral Oil Saturated Hydrocarbons" (Gesättigte Mineralölkohlenwasserstoffe)) oder MOAH (steht für Englisch "Mineral Oil Aromatic Hydrocarbons" (Aromatische Mineralölkohlenwasserstoffe)) zu enthalten. Dieses kann jedoch je nach Gewinnungsart ausgeschlossen werden, so dass eigentlich keine Verunreinigungen vorliegen. Dennoch meiden Verbraucher zunehmend auch unbedenkliche Produkte enthaltend Mineralöle.

Weiterhin sind aus dem Stand der Technik eine Vielzahl an kommerziellen schweisshemmenden Aerosolsprays bekannt, welche gemäß Produktauslobung geringere Textilanschmutzungen aufweisen sollen.

Der Umstand, dass bereits eine Vielzahl an Ansätzen bekannt ist, Textilverschmutzungen bei antitranspirant wirksamen Aerosol-Suspensionsprays zu reduzieren, sollte nicht darüber hinwegtäuschen, dass weiterhin Textilverschmutzungen durch antitranspirant wirksame Aerosol-Suspensionsprays weiter reduziert werden sollten.

Eine Aufgabe der vorliegenden Erfindung war es somit antitranspirant wirksame Aerosol-Suspensionsprays bereitzustellen, welche geringere Textilanschmutzungen verursachen, wobei auf den Einsatz von Silikonölen und Mineralölen verzichtet wird.

Gegenstand der vorliegenden Erfindung ist ein kosmetisches Produkt aufweisend
a) Einen Druckbehälter aufweisend ein Ausgabeventil geeignet zur Sprühapplikation;
b) Eine silikon-, wasser- und mineralöl-freie kosmetische Suspension umfassend, bezogen auf das Gesamtgewicht der Suspension,
   i. einen aluminiumhaltigen Antitranspirantwirkstoff,
   ii. 30 bis 40 Gew.-% Isopropyl Palmitate, und
   iii. zusätzlich insgesamt 15 bis 40 Gew.-% einer Ölmischung aufweisend mindestens ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m,
c) Ein Treibmittel enthaltend mindestens ein Gas gewählt aus der Gruppe Butan, Isobutan, Propan, Pentane und/oder Stickstoff,
dadurch gekennzeichnet, dass sowohl die kosmetische Suspension als auch das Treibmittel in dem Druckbehälter enthalten sind, wobei das Gewichtsverhältnis von der Suspension zum Treibmittel von 5:95 bis 60:40 beträgt.

Erfindungsgemäß ist das Isopropyl Palmitate nicht der Ölmischung iii. zuzurechnen.

Ein weiterer Gegenstand der Erfindung ist die Verwendung von 30 bis 40 Gew.-% Isopropyl Palmitate in Kombination mit 15 bis 40 Gew.-% einer Ölmischung aufweisend mindestens ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m in einer silikon-, wasser- und mineralöl-freie kosmetischen Suspension enthaltend einen aluminiumhaltigen Antitranspirantwirkstoff zur Reduzierung von Textilanschmutzungen verursacht von aluminiumhaltigen Antitranspirantwirkstoffen, wobei sich die vorstehenden Gewichtsangeben auf das Gesamtgewicht der Suspension beziehen.

Erfindungsgemäß ist in der Ölmischung kein Mineralöl oder Silikonöl enthalten. Der Ölmischung sind alle enthaltenen Öle zuzuordnen mit Ausnahme von Isopropyl Palmitate.

Alle nachstehend angegebenen Gewichtsprozente (Gew.-%) basieren, sofern nicht anders angegeben, auf dem Gesamtgewicht der erfindungsgemäßen Suspension. Wenn Verhältnisse bestimmter Komponenten in der folgenden Beschreibung offenbart sind, beziehen sich diese Verhältnisse, sofern nicht anders angegeben, auf Gewichtsverhältnisse der Komponenten.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf die erfindungsgemäße Zubereitung sowie die erfindungsgemäße Verwendung als auch auf erfindungsgemäße Verfahren.

Sofern nicht anders angegeben, wurden alle Tests und Messungen unter "normalen Bedingungen / Normalbedingungen" durchgeführt. Der Begriff "normale Bedingungen / Normalbedingungen" bezieht sich auf 20 ° C, 1013 hPa und eine relative Luftfeuchtigkeit von 50%.

Der Begriff "Haut" bezieht sich ausschließlich auf die menschliche Haut.

Der Begriff "wasserfrei" ist im Sinne der vorliegenden Offenbarung so zu verstehen, dass kein freies Wasser der Suspension zugesetzt wurde. Kristallwasser, Hydratationswasser oder ähnlich molekular gebundenem Wasser kann enthalten sein, wobei der Anteil generell nicht mehr als 5 Gew.-% bezogen auf das Gesamtgewicht der Suspension, beträgt.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min⁻¹, verwendet.

Die Anwendung des kosmetischen Produkts bzw. der Suspension der Erfindung vermindert oder vermeidet die Fleckenbildung in oder auf der Kleidung und verbessert die Auswaschbarkeit von Flecken, die durch die Suspension selber mit verursacht wurden, aus der Kleidung.

Unter Verminderung bzw. Vermeidung der Fleckenbildung auf Textilien vor und insbesondere nach der Wäsche wird erfindungsgemäß der verringerte b-Wert verstanden, der photometrisch mittels der Farbmaßzahlen im CIE- L*a* b*-Farbraum ermittelt wird, und der gegenüber den b-Werten der Textilie, befleckt mit einer Zubereitung mit Antitranspirantmittel aber ohne erfindungsgemäße Ölmischung, gemessen wird. Durch ein Vergleichsversuch ist gezeigt worden, dass die Fleckenbildung des erfindungsgemäßen kosmetischen Produkts in oder auf der Kleidung grundsätzlich vermindert ist bzw. die Kleidung weniger gelblich verfleckt ist.

Unter Textilverfleckung werden Verfleckungen auf Kleidungstextilien, insbesondere im Achselbereich, verstanden. Es handelt sich um Flecken, die nach dem Tragen und/oder Waschen in der Kleidung verbleiben und mit zunehmendem Alter des Kleidungsstückes intensiver werden können. Diese Flecken sind nicht die als "Weißeln" bezeichneten Rückstände auf der Haut oder Kleidung zu verstehen. Diese weißen Rückstände sind in der Regel mechanisch (Bürsten) oder durch Waschen zu entfernen.

Es handelt sich erfindungsgemäß vielmehr um meist gelbliche Verfleckungen, die entstehen, wenn das kosmetische Produkt oder Bestandteile davon beim Schwitzen zusammen mit den Körpersekreten der Achsel auf die Textilie gelangt. Durch das Waschen der Textilie wird ein Teil dieser Ablagerungen ausgewaschen, ein anderer Teil verbleibt als Rückstand auf dem Textil.

Als Maß der Verbesserung bzw. Verminderung wird dabei der Unterschied zur Fleckenbildung bzw. dessen Auswaschung bei der Anwendung des erfindungsgemäßen Produkts bzw. Suspension und eines nicht erfindungsgemäßen Produkts bzw. Suspension definiert.

Erfindungsgemäß geht es insbesondere um die bekannte gelbliche Verfleckung von Antitranspirantien in oder auf der Kleidung, nachdem das Kleidungsstück gewaschen wurde. Erfindungsgemäß setzt hier auch der Lösungsgedanke der verbesserten Auswaschbarkeit an. Der gelb-Wert des Fleckes wird daher insbesondere über den b-Wert definiert, der photometrisch mittels der Farbmaßzahlen im CIE- L*a*b*-Farbraum ermittelt werden kann. Bevorzugt bezieht sich die erfindungsgemäße Verwendung der Fleckenvermeidung bzw. verbesserten Auswaschbarkeit auf Textilien, die Baumwolle enthalten oder aus Baumwolle bestehen.

Die erfindungsgemäße Suspension umfasst mindestens einen aluminiumhaltigen Antitranspirantwirkstoff. Dabei ist es vorteilhaft, wenn als Antitranspirantwirkstoff mindestens ein Aluminiumsalz enthalten ist. Dabei ist es weiterhin insbesondere vorteilhaft, wenn der Antitranspirantwirkstoff gewählt ist aus der Gruppe der Aluminiumsalze mit der Formel [Al₂(OH)ₘClₙ], wobei m+n=6.

Erfindungsgemäße Beispiele von erfindungsgemäßen Aluminiumsalzen sind:
- Aluminium-Salze wie Aluminiumchlorid AlCl₃, Aluminiumsulfat Al₂(SO₄)₃
- Aluminiumchloride der empirischen Summenformel [Al₂(OH)ₘClₙ], wobei m+n=6
- Aluminiumchlorhydrat [Al₂(OH)₃Cl] x H₂O (ACH)
   ∘ Standard Al-Komplexe: Locron P (Clariant), Locron L (Clariant), Micro-Dry (Reheis), ACH-331 (Summit), Aloxicoll PF 40 (Giulini).
   ∘ Aktivierte Al-Komplexe: Reach 501 (Reheis), AACH-324 (Summit), AACH-7171 (Summit), Aloxicoll P (Giulini), Aloxicoll SD100
- Aluminiumsesquichlorhydrat [Al₂(OH)_{4,5}Cl_{1,5}] x H₂O
   ∘ Standard Al-Komplexe: Aluminium Sesquichlorohydrate (Reheis), AACH-308 (Summit)
   ∘ Aktivierte Al-Komplexe: Reach 301 (Reheis)
- Aluminiumdichlorhydrat [Al₂(OH)₄Cl₂] x H₂O

Ferner ist es vorteilhaft, wenn als Antitranspirantwirkstoff mindestens ein Aluminium-Zirkonium-Salz enthalten ist. Vorteilhaft sind diese gewählt aus der Gruppe Aluminium/Zirkonium Trichlorohydrex Glycin ([Al₄Zr(OH)₁₃Cl₃] x H₂O x Gly), Aluminium/Zirkonium Tetrachlorohydrex Glycin ([Al₄Zr(OH)₁₂Cl₄] x H₂O x Gly), Aluminium/Zirkonium Pentachlorohydrex Glycin ([Al₈Zr(OH)₂₃Cl₅] x H₂O x Gly), Aluminium/Zirkonium Octachlorohydrex Glycin ([Al₈Zr(OH)₂₀Cl₈] x H₂O x Gly) und der Glycinfreie Aluminium/Zirkonium-Salze. Insbesondere bevorzugt ist Aluminium/Zirkonium Tetrachlorohydrex Glycin.

Es ist insbesondere vorteilhaft, wenn als Antitranspirantwirkstoff Aluminium Chlorhydrate und/oder Aluminium Sesquichlorohydrate enthalten sind.

Vorteilhaft beträgt der Gesamtanteil der aluminiumhalten Antitranspirantwirkstoffe in der Suspension von 15 Gew.-% bis 45 Gew.-%, bevorzugt von 17 bis 40 Gew.-% und insbesondere bevorzugt 18 bis 37 Gew.-% bezogen auf das Gesamtgewicht der Suspension.

Weiterhin ist es vorteilhaft, wenn der Gesamtanteil von Aluminium Chlorhydrate und/oder Aluminium Sesquichlorohydrate in der Suspension von 10 Gew.-% bis 45 Gew.-%, bevorzugt von 12 bis 41 Gew.-% und insbesondere bevorzugt 15 bis 37 Gew.-%, bezogen auf das Gesamtgewicht der Suspension, beträgt.

Sofern sowohl Aluminium Chlorhydrate als auch Aluminium Sesquichlorohydrate beträgt das Gewichtsverhältnis von Aluminium Chlorhydrate zu Aluminium Sesquichlorohydrate vorteilhaft von 2:1 bis 15:1, bevorzugt von 4:1 bis 10:1 und insbesondere bevorzugt von 6:1 bis 8,5:1.

Erfindungsgemäß enthält die Suspension 30 bis 40 Gew.-% Isopropyl Palmitate, bezogen auf das Gesamtgewicht der Suspension. Es ist bevorzugt, wenn der Anteil von Isopropyl Palmitate von 31 Gew.-% bis 39 Gew.-%, mehr bevorzugt von 32 Gew.-% bis 38,5 Ge.-% und insbesondere bevorzugt von 32,5 Gew.-% bis 38 Gew.-% beträgt, bezogen auf das Gesamtgewicht der Suspension.

Weiterhin umfasst die erfindungsgemäße Suspension 15 bis 40 Gew.-% einer Ölmischung aufweisend mindestens ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m, wobei sich die Angaben auf das Gesamtgewicht der Suspension beziehen. Vorteilhaft umfasst die erfindungsgemäße Suspension 20 bis 37 Gew.-% einer Ölmischung aufweisend mindesten ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m, wobei sich die Angaben auf das Gesamtgewicht der Suspension beziehen. Insbesondere vorteilhaft umfasst die erfindungsgemäße Suspension 25 bis 35 Gew.-% einer Ölmischung aufweisend mindesten ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m, wobei sich die Angaben auf das Gesamtgewicht der Suspension beziehen.

Es ist ferner vorteilhaft, wenn die Suspension frei ist von Ölen, welche eine Grenzflächenspannung zu Wasser bei 20°C von mehr als 35,0 mN/m aufweisen.

Weiterhin ist es vorteilhaft, wenn der Gesamtanteil der Öle, welche eine Grenzflächenspannung zu Wasser bei 20°C von mehr als 30,0 mN/m aufweisen, zu dem Gesamtanteil der Öle, welche eine Grenzflächenspannung zu Wasser bei 20°C von weniger als 30,0 mN/m aufweisen ausgenommen Isopropyl Palmitate, von 1:0,5 bis 1:4,5, bevorzugt von 1:1,2 bis 1:4 und insbesondere von 1:2 bis 1:3,5 beträgt.

Die folgende Tabelle gibt die Grenzflächenspannung verschiedener Öle gegen Wasser bei 20°C an.

| INCI | Grenzflächenspannung vs. Wasser bei 20°C in mN/m |
|---|---|
| Octyldodecanol | 28 |
| Caprylic/Capric Triglyceride | 25,5 |
| Dicaprylyl Ether | 34 |
| Isopropyl Palmitate | 28,9 |
| C12-15 Alkyl Benzoate | 28,6 |
| Triethyl Citrate | 4,3 |

Überraschend konnte durch die Anpassung der Grenzflächenspannungen der Öle gemäß der Erfindung eine deutliche Reduzierung der erfindungsgemäß beschriebenen Textilanschmutzung bzw. eine verbesserte Auswaschbarkeit erzielt werden.

Es ist erfindungsgemäß von Vorteil, wenn die Ölmischung als Öl aufweisend eine Grenzflächenspannung gegen Wasser bei 20°C von mehr als 30,0 mN/m mindestens Dicaprylyl Ether enthält.

Ferner ist es bevorzugt, wenn die Ölmischung dadurch gekennzeichnet ist, dass mindestens ein Öl enthalten ist, welches eine Grenzflächenspannung zu Wasser bei 20°C von 0,5 bis 15 mN/m, bevorzugt von 2 bis 10 mN/m und insbesondere bevorzugt von 3 bis 8 mN/m aufweist.

Erfindungsgemäß vorteilhafte Ölmischungen umfassen vorteilhaft Dicaprylyl Ether und eines oder mehre Öle gewählt aus der Gruppe Octyldodecanol, Caprylic/Capric Triglyceride, C12-15 Alkyl Benzoate, Isoamyl Laurate, Cetearyl Isononanoate, Ethylhexyl Cocoate, C12-13 Alkyl Lactate und Triethylcitrate. Weiterhin erfindungsgemäß vorteilhafte Ölmischungen umfassen vorteilhaft Dicaprylyl Ether und eines oder mehre Öle gewählt aus der Gruppe Octyldodecanol, Caprylic/Capric Triglyceride, C12-15 Alkyl Benzoate und Triethylcitrate.

Vorteilhaft sind neben Dicaprylyl Ether, Octyldodecanol, Caprylic/Capric Triglyceride, C12-15 Alkyl Benzoate und/oder Triethylcitrate weitere Öle nur in Anteilen bis maximal 10 Gew.-%, bezogen auf das Gesamtgewicht der Suspension, in der Ölmischung der Suspension enthalten, wobei 0 bis 0,5 Gew.-% der weiteren Öle bevorzugt ist.

Sofern die Suspension der Erfindung Dicaprylyl Ether enthält, so ist es vorteilhaft, wenn der Anteil von Dicaprylyl Ether von 2 bis 25 Gew.-%, bevorzugt von 6 bis 13 Gew.-% und insbesondere bevorzugt von 8 bis 12 Gew.-%, bezogen auf das Gesamtgewicht der Suspension beträgt.

Sofern die Suspension der Erfindung Octyldodecanol enthält, so ist es vorteilhaft, wenn der Anteil von Octyldodecanol von 1 bis 25 Gew.-%, bevorzugt von 4 bis 10 Gew.-% und insbesondere bevorzugt von 6 bis 8 Gew.-%, bezogen auf das Gesamtgewicht der Suspension beträgt.

Sofern die Suspension der Erfindung Caprylic/Capric Triglyceride enthält, so ist es vorteilhaft, wenn der Anteil von Caprylic/Capric Triglyceride von 1 bis 25 Gew.-%, bevorzugt von 4 bis 10 Gew.-% und insbesondere bevorzugt von 6 bis 8 Gew.-%, bezogen auf das Gesamtgewicht der Suspension beträgt.

Sofern die Suspension der Erfindung C12-15 Alkyl Benzoate enthält, so ist es vorteilhaft, wenn der Anteil von C12-15 Alkyl Benzoate von 1 bis 25 Gew.-%, bevorzugt von 4 bis 10 Gew.-% und insbesondere bevorzugt von 6 bis 8 Gew.-%, bezogen auf das Gesamtgewicht der Suspension beträgt.

Sofern die Suspension der Erfindung Triethylcitrate enthält, so ist es vorteilhaft, wenn der Anteil von Triethylcitrate von 1 bis 25 Gew.-%, bevorzugt von 4 bis 10 Gew.-% und insbesondere bevorzugt von 6 bis 8 Gew.-%, bezogen auf das Gesamtgewicht der Suspension beträgt.

Generell sind Ausführungsformen bevorzugt, wenn der Gesamtanteil der Öle ausgenommen Isopropyl Palmitate aufweisend eine Grenzflächenspannung von mehr als 20 mN/m zu dem Gesamtanteil der Öle mit einer Grenzflächenspannung von 0,5 bis 15 mN/m bei 20°C gegen Wasser von 50:1 bis 1:1, bevorzugt von 20:1 zu 4:1 und insbesondere bevorzugt von 10:1 bis 7:1 beträgt.

Generell ist es in dieser Ausführungsform bevorzugt, wenn der Gesamtanteil der Öle ausgenommen Isopropyl Palmitate aufweisend eine Grenzflächenspannung von mehr als 20 mN/m zu dem Gesamtanteil der Öle mit einer Grenzflächenspannung von 2 bis 10 mN/m bei 20°C gegen Wasser von 50:1 bis 1:1, bevorzugt von 20:1 zu 4:1 und insbesondere bevorzugt von 10:1 bis 7:1 beträgt.

Generell ist es in dieser Ausführungsform bevorzugt, wenn der Gesamtanteil der Öle ausgenommen Isopropyl Palimitate aufweisend eine Grenzflächenspannung von mehr als 20 mN/m zu dem Gesamtanteil der Öle mit einer Grenzflächenspannung von 3 bis 8 mN/m bei 20°C gegen Wasser von 50:1 bis 1:1, bevorzugt von 20:1 zu 4:1 und insbesondere bevorzugt von 10:1 bis 7:1 beträgt.

Weitere Ausführungsformen der Erfindung sind dadurch gekennzeichnet, dass die Ölmischung mindestens ein Öl umfasst welches eine Alkylkette mit 8 Kohlenstoffatomen aufweist. Bekannte vorteilhafte Öle der Ölmischung aufweisend eine Alkylkette mit 8 Kohlenstoffatomen sind gewählt aus Dicaprylyl Ether, Caprylic/Capric Triglyceride, Coco-Caprylate/ Caprate und Propylheptyl Caprylate. Insbesondere vorteilhaft sind Dicaprylyl Ether und Caprylic/Capric Triglyceride in der Ölmischung enthalten.

Innerhalb dieser Ausführungsform ist es besonders bevorzugt, wenn die Öle der Ölmischung so gewählt sind, dass mindestens 8 Gew.-%, bevorzugt mindestens 13 Gew.-% und insbesondere bevorzugt mindestens 17 Gew.-% der Öle, bezogen auf das Gesamtgewicht der Ölmischung ohne Isopropyl Palmitate, eine Alkylkette mit 8 Kohlenstoffatomen aufweisen.

Weiterhin sind vorteilhafte Ausführungsformen der Erfindung dadurch gekennzeichnet, dass kein C12-15 Alkyl Benzoate enthalten ist. Folglich beträgt der Anteil von C12-15 Alkyl Benzoate 0 Gew.-%, bezogen auf das Gesamtgewicht der Suspension.

Weiterhin ist es vorteilhaft, wenn pflanzliche Öle in Anteilen von 0 bis maximal 1 Gew.-%, bezogen auf das Gesamtgewicht der Suspension, enthalten sind. Es ist insbesondere vorteilhaft, wenn pflanzliche Öle in einem Anteil von 0 Gew.-% bis 0,15 Gew.-%, bezogen auf das Gesamtgewicht der Suspension, enthalten sind.

Ferner ist es erfindungsgemäß von Vorteil, wenn die Suspension der Erfindung ein Schichtsilikat, bevorzugt Disteardimonium Hectorite umfasst. Ist ein Schichtsilikat enthalten, so ist es bevorzugt, wenn der Anteil von dem Schichtsilikat von 0,5 Gew.-% bis 5 Gew.-%, bezogen auf das Gesamtgewicht der Suspension, beträgt. Ist Disteardimonium Hectorite enthalten, so ist es bevorzugt, wenn der Anteil von Disteardimonium Hectorite von 0,5 Gew.-% bis 5 Gew.-%, bezogen auf das Gesamtgewicht der Suspension, beträgt.

Weiterhin ist es vorteilhaft, wenn die Zubereitung Propylene Carbonate enthält, wobei der Anteil von Propylene Carbonate bevorzugt von 0,3 bis 1,2 Gew.-%, bezogen auf das Gesamtgewicht der Suspension, beträgt.

Weiterhin ist es vorteilhaft, wenn die Suspension zusätzlich mindestens ein oder mehrere weitere unter Normalbedingungen flüssige Parfumstoffe enthält. Vorteilhaft gewählte Parfümstoffe sind ausgewählt aus ätherischen Ölen, Methyl dihydrojasmonate, Phenethyl alcohol, Linalyl acetate,1,3,4,6,7,8-Hexahydro-4,6,6,7,8,8-hexamethylcyclopenta-gamma-2-benzopyran, Methyl cedryl ketone, Hexyl salicylate, 4-t-Butylcyclohexyl acetate, Tetrahydrolinalool, Orange terpenes, Heliotropin, Terpinyl acetate, omega-Pentadecalactone, Methyl-alpha-ionone, Lavandin oil, Lemon oil, 7-Acetyl-1,1,3,4,4,6-hexamethyltetralin, Coumarin, Amyl salicylate, 2-tert-Pentyl-cyclohexyl acetate, 3-Methyl-5-phenyl-1-pentanol, Cedrol, Benzyl benzoate, Cashmeran, hexyl cinnamal, Butylphenyl Methylpropional, 4-(4-Hydroxy-4-methylpentyl)-3-cyclohexene-1-carboxyaldehyde, Benzyl salicylate, Hydroxycitronellal, Benzyl benzoate, Amyl Cinnamal, Limonene, Benzyl Alcohol, Eugenol, Isoeugenol, Cinnamyl Alcohol, Farnesol, Cinnamal, Anise Alcohol, Evernia Prunastri (Extract), Benzyl Cinnamate, Methyl 2-Octynoate, Amylcinnamyl Alcohol, Evernia Furfuracea (Extract), Linalool, 2,6-Dimethyl-7-octen-2-ol, Dipropylene glycol, decanal /aldehyde c-10, Ethylvanilin, 2-Acetonapthone-1,2,3,4,5,6,7,8-octahydro-2,3,8,8-tetramethyl, Butylphenyl Methylpropional, Benzyl acetate, Ethylene brassylate, Orange oil, alpha-Isomethyl lonone, Patchouli oil, Geraniol, Citronellol, Heliotropin, Bergamot oil, Ethyllinalool, Benzyl benzoate, Vanillin, 2-Isobutyl-4-hydroxy-4-methyltetrahydropyran, Terpineol, Lavender oil, Citral, Eugenol, Hexenol cis 3, Anisealdehyde, Nerol, 3-(4-tert-butylphenyl)propanal, Vetiver Essential Oil, Geranium Öl, 2-Ethyl-4-(2',2',3'-trimethylcyclopent-3'-enyl)-but-2-enol, 2-(2'-Methylpropyl)-4-hydroxy-4-methyltetrahydropyran, 3-Hexenylacetate, Rose Oxide, Ambroxan, Bourgeonal , Silvial , Cyclemax, Florhydral, Floralozone, Hydratropic Aldehyde, Methyl-N methylanthranilate, Styrax, Celestolide, Phantolide, Tonalide, Traseolide, Galaxolide, Camphor, Eucalyptol, Menthol, Cyclamen aldehyde, Adipinsäurediester, alpha-Amylcinnamaldehyde, alpha-Methylionon Benzoin, Hydroxy-3-Cyclohexencarboxaldehyde, Methylbenzoate, Muskatnussöl und Rosmarinöl. Weitere Parfümkomponenten, die enthalten sein können, aber nicht ausdrücklich bevorzugt werden, sind in Arctander, Perfume and Flavor Chemicals (Chemicals)Vol. I und II (1969) und Arctander, Perfume and Flavour Materials of Natural Origin (1960) genannt.

Vorteilhaft beträgt der Anteil der weiteren unter Normalbedingungen flüssigen Parfumstoffe von 0,001 bis 10 Gew.-%, bevorzugt von 0,005 bis 9 Gew.-% und insbesondere bevorzugt von 0,01 bis 8 Gew.-% bezogen auf das Gesamtgewicht der Suspension.

Weiterhin ist es vorteilhaft, wenn der die Suspension Palmitamidopropyltrimonium Chloride enthält. Ist Palmitamidopropyltrimonium Chloride enthalten, ist es vorteilhaft, wenn der Anteil von Palmitamidopropyltrimonium Chloride von 0,01 bis 5 Gew.-%, bevorzugt von 0,05 bis 0,5 Gew.-% bezogen auf das Gesamtgewicht der Suspension, beträgt.

Weiterhin ist es erfindungsgemäß möglich, dass die Zubereitungen weitere Hilfsstoffe enthalten. Diese können Stabilisatoren, Pflegestoffe, Deowirker oder weitere Inhaltstoffe umfassen.

Der Druckbehälter des kosmetischen Produkts ist bevorzugt dadurch gekennzeichnet, dass dieser aus Aluminium und/oder Aluminiumlegierungen gefertigt ist. Entsprechend umfasst die Zusammensetzung des Druckbehälters bevorzugt Aluminium und/oder Aluminiumlegierungen. Vorteilhaft ist dabei der Einsatz von recyceltem Aluminium.

Problematisch ist der Umstand, dass Druckbehälter aufweisend recyceltes Aluminium abhängig vom Recycling- oder Aufreinigungsverfahren Verunreinigungen aufweisen können, welche mit Bestandteilen der Formel so interagieren, dass es zu stärkeren Textilanschmutzungen oder weiteren Produktnachteilen kommen könnte. Möglich und denkbar sind beispielsweise Verunreinigungen durch Eisenionen und/oder Farbstoffe. Es ist vorstellbar, dass sich Bestandteile der Dose in der Suspension der Erfindung lösen.

Bekannt ist der Einsatz von Lacken zur Verhinderung der Interaktion des Wandmaterials der Druckbehälter mit der erfindungsgemäßen Suspension.

Überraschend wurde nun gefunden, dass Lacke verwendet werden können, denen kein BPA zugesetzt wurde. Vorteilhaft werden BPA freie Lacke eingesetzt, die auf Polyestern basierte sind. Der Druckbehälter weist entsprechend vorteilhaft eine Zusammensetzung auf, die Aluminium und/oder Aluminiumlegierungen umfasst, wobei der Druckbehälter auf der innenliegenden Fläche lackiert ist und der aufgetragene Lack Polyester umfasst und kein zugesetztes BPA aufweist. Es werden bevorzugt keine Epoxide basierten Lacke eingesetzt.

In Bezug auf die vorliegende Erfindung wurde die folgende In-vitro Methode angewendet, um die Textilverschmutzung zu prüfen.

### In vitro Methode zur Bestimmung der Fleckenintensität

### Materialien:

Textil: weiß, 100% Baumwolle

### Testsubstanzen/Medien

a.) Sebum: Sebum nach BEY
b.) Schweiß: Humanschweiß oder synthetischer Schweiß künstl. Achselschweiß (gemäß DIN 53160-2:2010-10)
c.) Vergleichsprodukte

Das Auftragen des Schweißes (b.)) ist optional.
Wasserqualität: Leitungswasser (vorzugsweise mittelhart bis hart)
Waschmittel: handelsübliche Waschmittel, z. B. Spee Megaperls
Waschmaschine: handelsübliche Waschmaschine, z. B. Miele

Das Sebum bewirkt, dass der Fleck einen vergleichbaren Gelbanteil bekommt. Ohne Sebum werden die Flecken eher grau. Eine derartige Methode ist in der Literatur bereits beschrieben: The Trouble with Stains. SPC July page 25-28; Materialien wie Sebum nach BEY sind beispielsweise als Standard-Testschmutz bei der WfK-Testgewebe GmbH erhältlich.

### Auftragsmengen:

Produkt: möglicher Bereich: 10 mg/cm² bis 50 mg/cm², vorzugsweise 13 mg/cm² bis 40 mg/cm², besonders bevorzugt 15 mg/cm² bis 30 mg/cm²
Sebum: möglicher Bereich: 1 mg/cm² bis 15 mg/cm², vorzugsweise 2 mg/cm² bis 10 mg/cm² besonders bevorzugt 3 mg/cm² bis 6 mg/cm²
Schweißlösung : möglicher Bereich: 5 mg/cm² bis 40 mg/cm², vorzugsweise 7 mg/cm² bis 30 mg/cm²
Verhältnis Produkt zu Sebum 1:1 bis 7:1, vorzugsweise 2:1 bis 6:1
Verhältnis Produkt zu Humanschweiß 1:3 bis 7:1, vorzugsweise 1:1 bis 4:1

### Farbmessung:

photometrisch, Ermittlung der Farbmaßzahlen im CIE- L*a*b*-Farbraum Differenzbildung gegenüber einem unverfleckten Referenzmaterial .
Mittelwert aus mind. 5 Messungen pro Areal .
Zur Auswertung wird vorzugsweise der db-Wert verwendet (Gelbwert) genutzt.

### Ablauf:

1. Vorwaschen und Trocknen der Textilzuschnitte
2. Kennzeichnung der Areale
3. Bevorzugtes Auftragen der Medien in folgender Reihenfolge: a.) Zubereitung, b.) ggf. Schweiß, c.) Sebum, nach jedem Material mind. 10 min warten, bis es eingezogen ist. Alternativ können die Substanzen vollständig oder teilweise vorab miteinander vermischt werden und dann in dieser Mischung aufgetragen werden.
4. Einlagern der Proben bei 38°C und 80% Luftfeuchte vorzugsweise für mind. 12 Stunden
5. Waschen in Waschmaschine bei 60°C Waschprogramm für Baumwolle, Waschmitteldosierung gemäß Herstellerangaben für mittlere Wasserhärte und normalen Verschmutzungsgrad
6. Trocknen, z. B bei Raumtemperatur, 40°C oder in einem handelsüblichen Trockner
7. Farbmessung auf den verfleckten Arealen und einem unverfleckten Referenzareal und Differenzbildung
8. Wiederholung des Ablaufes von Punkt 2 bis 7 mindestens 1 mal, vorzugsweise 4 mal
photometrisch, Ermittlung der Farbmaßzahlen im CIE- L*a*b*-Farbraum. Insbesondere Ermittlung des b-Werts gegenüber unbeflecktem Textil. Die Differenz ergibt den db*-Wert. Umso höher der db*-Wert, desto mehr Textilverfleckungen liegen vor.

### Methode zur Durchführung der Messung der Grenzflächenspannung.:

Die Krafttensiometermessungen der Öle gegen Wasser wurden mit den folgenden Parameter/Messinstrumenten durchgeführt:

| Messgerät: | Tensiometer K100 (KRÜSS) |
|---|---|
| Korrektur: | ja: Harkins & Jordan |
| Temperatur: | 20°C |

### Einstellungen:

| Messkörper: | Krüss Standard Ring (De Noüy) |
|---|---|
| Radius: | 9,545 mm |
| Drahtdurchmesser: | 0,37 mm |

### Parameter:

| | |
|---|---|
| Geschwindigkeitsoberflächenerkennung: | 4 mm/min |
| Sensitivität Oberfläche - Erkennung: | 0,001 g |
| Eintauchtiefe: | 3,00 mm |
| Werte: | 5 - 30 |
| Datenaufnahme: | linear |

Die Dichtemessungen für die Korrektur der Krafttensiometermessung wurden unter folgenden Bedingungen durchgeführt:

| | | |
|---|---|---|
| Methode: | | Dichte(min. triple determination) |
| Instrument: | | DMA 4500 |
| Korrektur: | | ja: für Viskositätenten < 700 mPas |
| System: | | flexural resonator |
| Temperature: | 20°C | |

### Beispiele:

Die folgenden Beispiele sollen die Produkte dieser Erfindung veranschaulichen, ohne die Erfindung auf diese Beispiele beschränken zu wollen. Die Zahlenwerte in den Beispielen sind Gewichtsprozente, bezogen auf das Gesamtgewicht der Suspensionen.

Die Werte für die Gelbverfleckung wurden gemäß folgendem Vorgehen ermittelt:
1. Vorwaschen und Trocknen der Textilzuschnitte aus Baumwollgewebe
2. Kennzeichnung der Areale
3. Mischen von Suspension und Sebum im Verhältnis 5:1
4. Auftragen von ca. 0,3 g dieser Mischung auf eine Fläche von ca. 12 cm²
5. Einlagern der Proben bei 38°C und 80% Luftfeuchte vorzugsweise für mind. 12 Stunden
6. Waschen in Waschmaschine bei 60°C Waschprogramm für Baumwolle, Waschmitteldosierung gemäß Herstellerangaben für mittlere Wasserhärte und normalen Verschmutzungsgrad, verwendetes Waschmittel Spee Mega Pearls
7. Trocknen bei 40°C Raumtemperatur für 1h
8. Dreimalige Wiederholung des Ablaufes von Punkt 4 bis 7
9. Farbmessung auf den verfleckten Arealen und einem unverfleckten Referenzareal und Differenzbildung mittels einem Gerät der Firma Datacolor, und zwar Datacolor 800V. Zur Auswertung werden die db*-Werte herangezogen.

Die nachfolgend mit der Abkürzung Vgl.X, wobei X eine fortlaufende Zahl ist, gekennzeichneten Suspensionen sind nicht erfindungsgemäße Vergleichsbeispiele. Erfindungsgemäße Suspensionen werden mit Bsp.X abgekürzt, wobei auch hier das X für eine fortlaufende Zahl steht.

| **Inhaltstoffe** | **Bsp.1** | **Vgl.1** | **Vgl.2** | **Vgl.3** | **Vgl.4** | **Vgl.5** |
|---|---|---|---|---|---|---|
| Aluminum Chlorohydrate | 20 | 20 | 20 | 20 | 20 | 20 |
| Parfum | 6,33 | 6,33 | 6,33 | 6,33 | 6,33 | 6,33 |
| Propylene Carbonate | 0,8 | 0,8 | 0,8 | 0,8 | 0,8 | 0,8 |
| Disteardimonium Hectorite | 4 | 4 | 4 | 4 | 4 | 4 |
| Palmitamidopropyltrimonium Chloride | 0,06 | 0,06 | 0,06 | 0,06 | 0,06 | 0,06 |
| Propylene Glycol | 0,04 | 0,04 | 0,04 | 0,04 | 0,04 | 0,04 |
| Isopropyl Palmitate | 37,77 | 68,77 | 0 | 0 | 0 | 0 |
| Octyldodecanol | 7 | 0 | 68,77 | 0 | 0 | 0 |
| Dicaprylyl Ether | 10 | 0 | 0 | 68,77 | 0 | 0 |
| Caprylic/Capric Triglyceride | 7 | 0 | 0 | 0 | 68,77 | 0 |
| Triethyl Citrate | 7 | 0 | 0 | 0 | 0 | 68,77 |
| | | | | | | |
| db*-Werte gegenüber unbeflecktem Textil | 3,57 | 4,17 | 3,87 | 3,81 | 3,85 | 3,78 |

Die oben genannten Suspensionen wurden wie folgt in eine Aerosoldose mit Ausgabeventil abgefüllt: 15 Gew.-% Suspension + 85 Gew.-% Treibgas (Gemisch aus Propan, Isobutan, Butan). Die Aerosoldosen, bzw. Druckbehälter, weisen ein Ausgabeventil auf, was per Hand so betätigt werden kann, dass die Suspension versprüht wird. Der Druckbehälter umfasst Aluminium, eine Aluminiumlegierung und/oder recyceltes Aluminium und ist innen mit einem Schutzlack versehen, der kein zugesetztes BPA aufweist.

Es zeigt sich, dass überraschend durch den Einsatz der erfindungsgemäßen Ölmischung, die Textilanschmutzung nach dem Waschen deutlich reduziert wird.

Ein zweiter Vergleichsversuch zeigt die vorteilhafte Wirksamkeit im Vergleich zu Suspensionen aufweisend Silikonöle (Vgl.6 und Vgl.7). Bsp.2 und Bsp.3 sind erfindungsgemäße Beispiele. Für diesen Vergleichsversuch wurden die folgenden Suspensionen bereitgestellt:

| | Vgl. 6 | Bsp. 2 | Vgl. 7 | Bsp. 3 |
|---|---|---|---|---|
| Aluminum Sesquichlorohydrate | 0 | 0 | 4,38 | 5 |
| Aluminum Chlorohydrate | 16,67 | 20 | 35 | 35 |
| Triethyl Citrate | 0 | 7 | 0 | 0 |
| Palmitamidopropyltrimonium Chloride | 0,06 | 0,06 | 0 | 0 |
| Propylene Glycol | 0,04 | 0,04 | 0 | 0 |
| Cyclomethicone | 52,38 | 0 | 35,42 | 0 |
| Dimethiconol | 0,75 | 0 | 0,45 | 0 |
| | 0,1 | 0,1 | 0 | 0 |
| Dimethicone | 0 | 0 | 3 | 0 |
| Isopropyl Palmitate | 10 | 38,27 | 10 | 31,25 |
| C12-15 Alkyl Benzoate | 10 | 7 | 0 | 7 |
| Octyldodecanol | 0,8 | 7 | 0,8 | 0,8 |
| Dicaprylyl Ether | 0 | 10 | 0 | 10 |
| Persea Gratissima Oil | 0 | 0 | 0,1 | 0,1 |
| Parfum | 6,4 | 6,33 | 6,6 | 6,6 |
| Propylene Carbonate | 0,8 | 0,7 | 0,7 | 0,7 |
| Disteardimonium Hectorite | 2 | 3,5 | 3,5 | 3,5 |
| Magnesium Aluminum Silicate | 0 | 0 | 0,05 | 0,05 |
| | | | | |
| db* gegenüber unverflecktem Textil | 3,45 | 3,03 | 3,83 | 3,33 |

Die oben genannten Suspensionen wurden wie folgt in eine Aerosoldose mit Ausgabeventil abgefüllt: 15 Gew.% Suspension + 85 Gew.% Treibgas (Gemisch aus Propan, Isobutan, Butan). Die Aerosoldosen, bzw. Druckbehälter, weisen ein Ausgabeventil auf, was per Hand so betätigt werden kann, dass die Suspension versprüht wird. Der Druckbehälter umfasst Aluminium, eine Aluminiumlegierung und/oder recyceltes Aluminium und ist innen mit einem Schutzlack versehen, der kein zugesetztes BPA aufweist.

Weitere Beispiele von Suspensionen gemäß der Erfindung sind in der folgenden Tabelle zusammengefasst.

| **Inhaltsstoffe** | **Bsp.4** | **Bsp.5** | **Bsp.6** | **Bsp.7** | **Bsp.8** |
|---|---|---|---|---|---|
| Aluminum Chlorohydrate | 35 | | 25 | 20 | 23,33 |
| Aluminum Sesquichlorohydrate | 5 | 23,33 | | | |
| Parfum | 6,6 | 6,33 | 6,4 | 6,4 | 6,5 |
| Triethyl Citrate | | 7 | | 6 | |
| Dicaprylyl Ether | 10 | 10 | 9 | 6 | 12 |
| Isopropyl Palmitate | 33,05 | 35,04 | 39,7 | 39,4 | 37,77 |
| C12-15 Alkyl Benzoate | | 7 | | 6 | |
| Caprylic/Capric Trigylceride | 7 | | 8 | 6 | 8 |
| Octyldodecanol | 0,8 | 7 | 7 | 6 | 8 |
| Propylene Carbonate | 0,4 | 0,7 | 0,8 | 0,7 | 0,7 |
| Disteardimonium Hectorite | 2 | 3,5 | 4 | 3,5 | 3,5 |
| Avocadooil | 0,1 | | | | 0,1 |
| Magnesium Aluminum Silicate | 0,05 | | | | |
| Palmitamidopropyltrimonium Chlorid | | 0,06 | 0,06 | | 0,06 |
| Propylene Glycol | | 0,04 | 0,04 | | 0,04 |

Die oben genannten Suspensionen wurden wie folgt in eine Aerosoldose mit Ausgabeventil abgefüllt: 15 Gew.% Suspension + 85 Gew.% Treibgas (Gemisch aus Propan, Isobutan, Butan), 30 Gew.% Suspension + 70 Gew.% Treibgas (Gemisch aus Propan, Isobutan, Butan) und 40 Gew.% Suspension + 60 Gew.% Treibgas (Gemisch aus Propan, Isobutan, Butan). Die Aerosoldosen, bzw. Druckbehälter, weisen ein Ausgabeventil auf, was per Hand so betätigt werden kann, dass die Suspension versprüht wird. Der Druckbehälter umfasst Aluminium, eine Aluminiumlegierung und/oder recyceltes Aluminium und ist innen mit einem Schutzlack versehen, der kein zugesetztes BPA aufweist.

## Patentansprüche

1. Kosmetisches Produkt aufweisend
a) Einen Druckbehälter aufweisend ein Ausgabeventil geeignet zur Sprühapplikation;
b) Eine silikon-, wasser- und mineralöl-freie kosmetische Suspension umfassend, bezogen auf das Gesamtgewicht der Suspension,
i. einen aluminiumhaltigen Antitranspirantwirkstoff,
ii. 30 bis 40 Gew.-% Isopropyl Palmitate, und
iii. zusätzlich insgesamt 15 bis 40 Gew.-% einer Ölmischung aufweisend mindestens ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m,
c) Ein Treibmittel enthaltend mindestens ein Gas gewählt aus der Gruppe Butan, Isobutan, Propan, Pentane und/oder Stickstoff,
**dadurch gekennzeichnet, dass** sowohl die kosmetische Suspension als auch das Treibmittel in dem Druckbehälter enthalten sind, wobei das Gewichtsverhältnis von der Suspension zum Treibmittel von 5:95 bis 60:40 beträgt.

2. Kosmetisches Produkt gemäß Anspruch 1 **dadurch gekennzeichnet, dass** der Antitranspirantwirkstoff gewählt ist aus der Gruppe der Aluminiumsalze mit der Formel [Al₂(OH)ₘClₙ], wobei m+n=6.

3. Kosmetisches Produkt nach einem der vorhergehenden Ansprüche **dadurch gekennzeichnet, dass** als Antitranspirantwirkstoff Aluminium Chlorhydrate und/oder Aluminium Sesquichlorohydrate enthalten sind.

4. Kosmetisches Produkt nach einem der vorhergehenden Ansprüche **dadurch gekennzeichnet, dass** der Gesamtanteil der aluminiumhalten Antitranspirantwirkstoffe in der Suspension von 15 Gew.-% bis 45 Gew.-%, bevorzugt von 17 bis 40 Gew.-% und insbesondere bevorzugt 18 bis 37 Gew.-% bezogen auf das Gesamtgewicht der Suspension beträgt.

5. Kosmetisches Produkt nach einem der vorhergehenden Ansprüche **dadurch gekennzeichnet, dass** der Anteil von Isopropyl Palmitate von 31 Gew.-% bis 39 Gew.-%, mehr bevorzugt von 32 Gew.-% bis 38,5 Ge.-% und insbesondere bevorzugt von 32,5 Gew.-% bis 38 Gew.-% beträgt, bezogen auf das Gesamtgewicht der Suspension.

6. Kosmetisches Produkt nach einem der vorhergehenden Ansprüche **dadurch gekennzeichnet, dass** die Suspension 25 bis 35 Gew.-% einer Ölmischung aufweisend mindesten ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m enthält, wobei sich die Angaben auf das Gesamtgewicht der Suspension beziehen.

7. Kosmetisches Produkt nach einem der vorhergehenden Ansprüche **dadurch gekennzeichnet, dass** die Suspension frei ist von Ölen, welche eine Grenzflächenspannung zu Wasser bei 20°C von mehr als 35,0 mN/m aufweisen.

8. Kosmetisches Produkt nach einem der vorhergehenden Ansprüche **dadurch gekennzeichnet, dass** der Gesamtanteil der Öle, welche eine Grenzflächenspannung zu Wasser bei 20°C von mehr als 30,0 mN/m aufweisen, zu dem Gesamtanteil der Öle, welche eine Grenzflächenspannung zu Wasser bei 20°C von weniger als 30,0 mN/m aufweisen ausgenommen Isopropyl Palmitate, von 1:0,5 bis 1:4,5, bevorzugt von 1:1,2 bis 1:4 und insbesondere von 1:2 bis 1:3,5 beträgt.

9. Kosmetisches Produkt nach einem der vorhergehenden Ansprüche **dadurch gekennzeichnet, dass** Ölmischung **dadurch gekennzeichnet ist, dass** mindestens ein Öl enthalten ist, welches eine Grenzflächenspannung zu Wasser bei 20°C von 0,5 bis 15 mN/m, bevorzugt von 2 bis 10 mN/m und insbesondere bevorzugt von 3 bis 8 mN/m aufweist.

10. Kosmetisches Produkt nach einem der vorhergehenden Ansprüche **dadurch gekennzeichnet, dass** die Ölmischung Dicaprylyl Ether und eines oder mehre Öle gewählt aus der Gruppe Octyldodecanol, Caprylic/Capric Triglyceride, C12-15 Alkyl Benzoate, Isoamyl Laurate, Cetearyl Isononanoate, Ethylhexyl Cocoate, C12-13 Alkyl Lactate und Triethylcitrate umfasst.

11. Kosmetisches Produkt nach einem der vorhergehenden Ansprüche **dadurch gekennzeichnet, dass** Dicaprylyl Ether in der Ölmischung enthalten ist und der Anteil von Dicaprylyl Ether von 2 bis 25 Gew.-%, bevorzugt von 6 bis 13 Gew.-% und insbesondere bevorzugt von 8 bis 12 Gew.-%, bezogen auf das Gesamtgewicht der Suspension, beträgt.

12. Kosmetisches Produkt nach einem der vorhergehenden Ansprüche **dadurch gekennzeichnet, dass** die Ölmischung Caprylic/Capric Triglyceride enthält, wobei der Anteil von Caprylic/Capric Triglyceride von 1 bis 25 Gew.-%, bevorzugt von 4 bis 10 Gew.-% und insbesondere bevorzugt von 6 bis 8 Gew.-%, bezogen auf das Gesamtgewicht der Suspension, beträgt.

13. Kosmetisches Produkt nach einem der vorhergehenden Ansprüche **dadurch gekennzeichnet, dass** die Ölmischung Triethylcitrate enthält, wobei der Anteil von Triethylcitrate von 1 bis 25 Gew.-%, bevorzugt von 4 bis 10 Gew.-% und insbesondere bevorzugt von 6 bis 8 Gew.-%, bezogen auf das Gesamtgewicht der Suspension, beträgt.

14. Kosmetisches Produkt nach einem der vorhergehenden Ansprüche **dadurch gekennzeichnet, dass** die Ölmischung Octyldodecanol enthält, wobei der Anteil von Octyldodecanol von 1 bis 25 Gew.-%, bevorzugt von 4 bis 10 Gew.-% und insbesondere bevorzugt von 6 bis 8 Gew.-%, bezogen auf das Gesamtgewicht der Suspension, beträgt.

15. Kosmetisches Produkt nach einem der vorhergehenden Ansprüche **dadurch gekennzeichnet, dass** die Ölmischung kein C12-15 Alkyl Benzoate umfasst.

16. Kosmetisches Produkt nach einem der vorhergehenden Ansprüche **dadurch gekennzeichnet, dass** die Ölmischung mindestens ein Öl umfasst welches eine Alkylkette mit 8 Kohlenstoffatomen aufweist, wobei es vorteilhaft ist, wenn die Öle der Ölmischung so gewählt sind, dass mindestens 25 Gew.-%, bevorzugt mindestens 35 Gew.-% und insbesondere bevorzugt mindestens 45 Gew.-% der Öle, bezogen auf das Gesamtgewicht der Ölmischung ohne Isopropyl Palmitate, eine Alkylkette mit 8 Kohlenstoffatomen aufweisen.

17. Verwendung von 30 bis 40 Gew.-% Isopropyl Palmitate in Kombination mit 15 bis 40 Gew.-% einer Ölmischung aufweisend mindestens ein Öl mit einer Grenzflächenspannung zu Wasser bei 20°C von mehr als 30,0 mN/m und mindestens ein weiteres Öl aufweisend eine Grenzflächenspannung zu Wasser bei 20°C von weniger als 30,0 mN/m in einer silikon-, wasser- und mineralöl-freie kosmetischen Suspension enthaltend einen aluminiumhaltigen Antitranspirantwirkstoff zur Reduzierung von Textilanschmutzungen verursacht von aluminiumhaltigen Antitranspirantwirkstoffen, wobei sich die vorstehenden Gewichtsangeben auf das Gesamtgewicht der Suspension beziehen.

## Claims

1. Cosmetic product having
a) a pressure vessel having a dispensing valve suitable for spray application;
b) a silicone-, water- and mineral oil-free cosmetic suspension comprising, based on the total weight of the suspension,
i. an aluminium-containing antiperspirant active ingredient,
ii. 30% to 40% by weight of Isopropyl Palmitate, and
iii. additionally a total of 15% to 40% by weight of an oil mixture having at least one oil with an interfacial tension with water at 20°C of more than 30.0 mN/m and at least one further oil having an interfacial tension with water at 20°C of less than 30.0 mN/m,
c) a propellant comprising at least one gas selected from the following group: butane, isobutane, propane, pentanes and/or nitrogen,
**characterized in that** both the cosmetic suspension and the propellant are present in the pressure vessel, where the weight ratio of the suspension to the propellant is from 5:95 to 60:40.

2. Cosmetic product according to Claim 1, **characterized in that** the antiperspirant active ingredient is selected from the group of aluminium salts with the formula [Al₂(OH)ₘClₙ], where m+n=6.

3. Cosmetic product according to either of the preceding claims, **characterized in that** Aluminium Chlorohydrate and/or Aluminium Sesquichlorohydrate are present as antiperspirant active ingredient.

4. Cosmetic product according to any of the preceding claims, **characterized in that** the total proportion of the aluminium-containing antiperspirant active ingredients in the suspension is from 15% by weight to 45% by weight, preferably from 17% to 40% by weight and especially preferably 18% to 37% by weight, based on the total weight of the suspension.

5. Cosmetic product according to any of the preceding claims, **characterized in that** the proportion of Isopropyl Palmitate is from 31% by weight to 39% by weight, more preferably from 32% by weight to 38.5% by weight and especially preferably from 32.5% by weight to 38% by weight, based on the total weight of the suspension.

6. Cosmetic product according to any of the preceding claims, **characterized in that** the suspension comprises 25% to 35% by weight of an oil mixture having at least one oil with an interfacial tension with water at 20°C of more than 30.0 mN/m and at least one further oil having an interfacial tension with water at 20°C of less than 30.0 mN/m, where the figures relate to the total weight of the suspension.

7. Cosmetic product according to any of the preceding claims, **characterized in that** the suspension is free of oils which have an interfacial tension with water at 20°C of more than 35.0 mN/m.

8. Cosmetic product according to any of the preceding claims, **characterized in that** the total proportion of the oils which have an interfacial tension with water at 20°C of more than 30.0 mN/m to the total proportion of the oils which have an interfacial tension with water at 20°C of less than 30.0 mN/m, except for Isopropyl Palmitate, is from 1:0.5 to 1:4.5, preferably from 1:1.2 to 1:4 and in particular from 1:2 to 1:3.5.

9. Cosmetic product according to any of the preceding claims, **characterized in that** the oil mixture is **characterized in that** at least one oil which has an interfacial tension with water at 20°C of 0.5 to 15 mN/m, preferably from 2 to 10 mN/m and especially preferably from 3 to 8 mN/m is present.

10. Cosmetic product according to any of the preceding claims, **characterized in that** the oil mixture comprises Dicaprylyl Ether and one or more oils selected from the following group: Octyldodecanol, Caprylic/Capric Triglyceride, C12-15 Alkyl Benzoate, Isoamyl Laurate, Cetearyl Isononanoate, Ethylhexyl Cocoate, C12-13 Alkyl Lactate and Triethyl Citrate.

11. Cosmetic product according to any of the preceding claims, **characterized in that** Dicaprylyl Ether is present in the oil mixture and the proportion of Dicaprylyl Ether is from 2% to 25% by weight, preferably from 6% to 13% by weight and especially preferably from 8% to 12% by weight, based on the total weight of the suspension.

12. Cosmetic product according to any of the preceding claims, **characterized in that** the oil mixture comprises Caprylic/Capric Triglyceride, where the proportion of Caprylic/Capric Triglyceride is from 1% to 25% by weight, preferably from 4% to 10% by weight and especially preferably from 6% to 8% by weight, based on the total weight of the suspension.

13. Cosmetic product according to any of the preceding claims, **characterized in that** the oil mixture comprises Triethyl Citrate, where the proportion of Triethyl Citrate is from 1% to 25% by weight, preferably from 4% to 10% by weight and especially preferably from 6% to 8% by weight, based on the total weight of the suspension.

14. Cosmetic product according to any of the preceding claims, **characterized in that** the oil mixture comprises Octyldodecanol, where the proportion of Octyldodecanol is from 1% to 25% by weight, preferably from 4% to 10% by weight and especially preferably from 6% to 8% by weight, based on the total weight of the suspension.

15. Cosmetic product according to any of the preceding claims, **characterized in that** the oil mixture does not comprise any C12-15 Alkyl Benzoate.

16. Cosmetic product according to any of the preceding claims, **characterized in that** the oil mixture comprises at least one oil which has an alkyl chain with 8 carbon atoms, it being advantageous if the oils of the oil mixture are selected such that at least 25% by weight, preferably at least 35% by weight and especially preferably at least 45% by weight of the oils, based on the total weight of the oil mixture without Isopropyl Palmitate, has an alkyl chain with 8 carbon atoms.

17. Use of 30% to 40% by weight of Isopropyl Palmitate in combination with 15% to 40% by weight of an oil mixture having at least one oil with an interfacial tension with water at 20°C of more than 30.0 mN/m and at least one further oil having an interfacial tension with water at 20°C of less than 30.0 mN/m in a silicone-, water- and mineral oil-free cosmetic suspension comprising an aluminium-containing antiperspirant active ingredient for reducing textile soiling caused by aluminium-containing antiperspirant active ingredients, where the above weight figures relate to the total weight of the suspension.

## Revendications

1. Produit cosmétique comprenant
a) un récipient sous pression comprenant une valve de distribution appropriée à l'application par pulvérisation ;
b) une suspension cosmétique exempte de silicone, d'eau et d'huile minérale, comprenant, par rapport au poids total de la suspension,
i. une substance active antitranspirante contenant de l'aluminium,
ii. 30 à 40 % en poids d'Isopropyl Palmitate, et
iii. en outre de 15 à 40 % en poids au total d'un mélange d'huiles comportant au moins une huile ayant une tension interfaciale avec l'eau à 20 °C de plus de 30,0 mN/m et au moins une autre huile présentant une tension interfaciale avec l'eau à 20 °C de moins de 30,0 mN/m,
c) un agent propulseur contenant au moins un gaz choisi dans le groupe constitué par le butane, l'isobutane, le propane, les pentanes et/ou l'azote,
**caractérisé en ce qu'**aussi bien la suspension cosmétique que l'agent propulseur sont contenus dans le récipient sous pression, le rapport en poids de la suspension à l'agent propulseur valant de 5:95 à 60:40.

2. Produit cosmétique selon la revendication 1, **caractérisé en ce que** la substance active antitranspirante est choisie dans le groupe des sels d'aluminium de formule [Al₂(OH)ₘClₙ], où m+n=6.

3. Produit cosmétique selon l'une quelconque des revendications précédentes, **caractérisé en ce que** de l'Aluminium Chlorhydrate et/ou des sesquichlorhydrates d'aluminium sont contenus en tant que substance active antitranspirante.

4. Produit cosmétique selon l'une quelconque des revendications précédentes, **caractérisé en ce que** la proportion totale des substances actives antitranspirantes contenant de l'aluminium dans la suspension vaut de 15 % en poids à 45 % en poids, de préférence de 17 à 40 % en poids, et de façon particulièrement préférée 18 à 37 % en poids par rapport au poids total de la suspension.

5. Produit cosmétique selon l'une quelconque des revendications précédentes, **caractérisé en ce que** la proportion d'Isopropyl Palmitate vaut de 31 % en poids à 39 % en poids, plus préférablement de 32 % en poids à 38,5 % en poids et de façon particulièrement préférée de 32,5 % en poids à 38 % en poids, par rapport au poids total de la suspension.

6. Produit cosmétique selon l'une quelconque des revendications précédentes, **caractérisé en ce que** la suspension contient de 25 à 35 % en poids d'un mélange d'huiles comportant au moins une huile ayant une tension interfaciale avec l'eau à 20 °C de plus de 30,0 mN/m et au moins une autre huile présentant une tension interfaciale avec l'eau à 20 °C de moins de 30,0 mN/m, les données se rapportant au poids total de la suspension.

7. Produit cosmétique selon l'une quelconque des revendications précédentes **caractérisé en ce que** la suspension est exempte d'huiles qui présentent une tension interfaciale avec l'eau à 20 °C de plus de 35,0 mN/m.

8. Produit cosmétique selon l'une quelconque des revendications précédentes, **caractérisé en ce que** la proportion totale des huiles qui présentent une tension interfaciale avec l'eau à 20 °C de plus de 30,0 mN/m, par rapport à la proportion totale des huiles qui présentent une tension interfaciale avec l'eau à 20 °C de moins de 30,0 mN/m, hormis l'Isopropyl Palmitate, vaut de 1:0,5 à 1:4,5, de préférence de 1:1,2 à 1:4 et en particulier de 1:2 à 1:3,5.

9. Produit cosmétique selon l'une quelconque des revendications précédentes, **caractérisé en ce que** le mélange d'huiles est **caractérisé en ce qu'**est contenue au moins une huile qui présente une tension interfaciale avec l'eau à 20 °C de 0,5 à 15 mN/m, de préférence de 2 à 10 mN/m et de façon particulièrement préférée de 3 à 8 mN/m.

10. Produit cosmétique selon l'une quelconque des revendications précédentes, **caractérisé en ce que** le mélange d'huiles comprend du Dicaprylyl Ether et une ou plusieurs huiles choisies dans le groupe constitué par l'Octyldodecanol, le Caprylic/Capric Triglyceride, le C12-15 Alkyl Benzoate, l'Isoamyl Laurate, le Cetearyl Isononanoate, l'Ethylhexyl Cocoate, le C12-13 Alkyl Lactate et le Triethylcitrate.

11. Produit cosmétique selon l'une quelconque des revendications précédentes, **caractérisé en ce que** du Dicaprylyl Ether est contenu dans le mélange d'huiles et la proportion totale de Dicaprylyl Ether vaut de 2 à 25 % en poids, de préférence de 6 à 13 % en poids, et de façon particulièrement préférée 8 à 12 % en poids, par rapport au poids total de la suspension.

12. Produit cosmétique selon l'une quelconque des revendications précédentes, **caractérisé en ce que** le mélange d'huiles contient du Caprylic/Capric Triglyceride, la proportion de Caprylic/Capric Triglyceride valant de 1 à 25 % en poids, de préférence de 4 à 10 % en poids, et de façon particulièrement préférée de 6 à 8 % en poids, par rapport au poids total de la suspension.

13. Produit cosmétique selon l'une quelconque des revendications précédentes, **caractérisé en ce que** le mélange d'huiles contient du Triethylcitrate, la proportion de Triethylcitrate valant de 1 à 25 % en poids, de préférence de 4 à 10 % en poids, et de façon particulièrement préférée de 6 à 8 % en poids, par rapport au poids total de la suspension.

14. Produit cosmétique selon l'une quelconque des revendications précédentes, **caractérisé en ce que** le mélange d'huiles contient de l'Octyldodecanol, la proportion d'Octyldodecanol valant de 1 à 25 % en poids, de préférence de 4 à 10 % en poids, et de façon particulièrement préférée de 6 à 8 % en poids, par rapport au poids total de la suspension.

15. Produit cosmétique selon l'une quelconque des revendications précédentes, **caractérisé en ce que** le mélange d'huiles ne comprend pas de C12-15 Alkyl Benzoate.

16. Produit cosmétique selon l'une quelconque des revendications précédentes, **caractérisé en ce que** le mélange d'huiles comprend au moins une huile qui comporte une chaîne alkyle ayant 8 atomes de carbone, où il est avantageux que les huiles du mélange d'huiles soient choisies de manière qu'au moins 25 % en poids, de préférence au moins 35 % en poids et de façon particulièrement préférée au moins 45 % en poids des huiles, par rapport au poids total du mélange d'huiles sans Isopropyl Palmitate, comportent une chaîne alkyle ayant 8 atomes de carbone.

17. Utilisation de 30 à 40 % en poids d'Isopropyl Palmitate en association avec 15 à 40 % en poids d'un mélange d'huiles comportant au moins une huile ayant une tension interfaciale avec l'eau à 20 °C de plus de 30,0 mN/m et au moins une autre huile ayant une tension interfaciale avec l'eau à 20 °C de moins de 30,0 mN/m dans une suspension cosmétique exempte de silicone, d'eau et d'huile minérale, contenant une substance active antitranspirante, pour la réduction de salissures textiles provoquées par des substances actives antitranspirantes contenant de l'aluminium, les données pondérales précédentes se rapportant au poids total de la suspension.
